# EUROPÄISCHE PATENTSCHRIFT

(11) **EP 0 831 765 B1**
(45) Veröffentlichungstag und Bekanntmachung des Hinweises auf die Patenterteilung: **11.04.2001**
(21) Anmeldenummer: 96917344.2
(22) Anmeldetag: 07.06.1996
(51) Int. Cl.: A61K 7/42

(54) **MEHRPHASIGES LICHTSCHUTZMITTEL, VERFAHREN ZUR HERSTELLUNG UND ZUM AUFTRAGEN AUF DIE HAUT**
MULTI-PHASE LIGHT SCREENING AGENT, PROCESS FOR ITS PRODUCTION AND FOR ITS APPLICATION TO THE SKIN
ECRAN SOLAIRE A PHASES MULTIPLES, PROCEDE DE PREPARATION ET D'APPLICATION SUR LA PEAU

(30) Priorität: 08.06.1995 DE 19521951
(43) Veröffentlichungstag der Anmeldung: 01.04.1998
(73) Patentinhaber: LANCASTER GROUP GmbH, 55116 Mainz (DE)
(72) Erfinder: GOLZ, Karin, 98000 Monaco (MC); ZASTROW, Leonhard, 98000 Monaco (MC); STANZL, Klaus, White Plains, NJ 10605 (US); GERBRON, Jaques, 06500 Menton (FR); FERRERO, Louis, 06000 Nice (FR)
(74) Vertreter: Walter, Wolf-Jürgen
(86) Internationale Anmeldenummer: DE9601046
(87) Internationale Veröffentlichungsnummer: WO9641613

(56) Entgegenhaltungen:
- EP-A- 0 619 999
- WO-A-94/17779
- WO-A-94/22419
- DE-C- 4 426 952

## Beschreibung

Die Erfindung betrifft ein neues mehrphasiges Lichtschutzmittel mit verbesserten Lichtschutzeigenschaften.

Es sind bereits eine Vielzahl von Lichtschutzformulierungen bekannt; die als Emulsionen für die Ausfilterung (Absorption oder Reflexion) der UVB-Strahlung (Bereich 290 bis 320 nm) und der UVA-Strahlung (Bereich 320 bis 400 nm) bestimmte organische und anorganische Wirkstoffe enthalten. Als organische Wirkstoffe sind insbesondere die Derivate des Dibenzoylmethans, des Benzophenons, der Salicylsäure, der Zimtsäure, des 3-Benzylidencamphers, der 4-Aminobenzoesäure usw. bekannt. Als anorganische Wirkstoffe sind Pigmente bekannt, wie die Oxide von Titanitim, Eisen, Zink; Silicium, Aluminium; Zirkonium usw. Ein dem Titaniumdioxid als Überlegen bezeichnetes Gemisch von organischen und anorganischen Stoffen ist ebenfalls bekannt (EP-A-588498).

Die WO94/22419 beschreibt ein Hautbräunungsmittel, bei dem zwei komponenten in getrennten Behältern vorliegen und beim Mischen auf der Haut miteinander reagieren und eine Farbbildung erzeugen.

In der Hauptsache ist die Entwicklung bei den Lichtschutzformulierungen zu neuen Wirkstoffen hingegangen bzw. zur Kombination von bekannten Wirkstoffen. Allgemein liegen diese in Emulsionen vor, um eine gute und gleichmäßige Verteilung auf der Haut zu gewährleisten, wobei Agglomerationen Von anorganischen Pigmenten durch grenzflächenaktive stoffe verhindert wurden.

Es sind jedoch auch Hydrodispersionen bekannt geworden, bei denen ein Emulgator nicht vorhanden ist, und die eine Dispersion einer diskontinuierlichen Lipidphase (flüssig, fest oder halbfest) in einer äußeren kontinuierlichen (wäßrigen) Phase darstellen. Die Stabilität eines solchen Emulgator-freien Systems wird z.B. durch Aufbau eines Gelgerüstes in der wäßrigen Phase erreicht mit einer stabilen Suspendierung der Lipidtröpfchen in diesem Gelgerüst. Nachteilig bei diesen Hydrodispersionen ist die notwendige hohe Konzentration von UV-Filtermaterialien und deren Klebrigkeit.

Die EP-A-603080 beschreibt eine kosmetische oder dermatologische Zwei-Phasen-Zusammensetzung, bestehend aus einer wäßrigen und einer separaten ölphase, die in der wäßrigen Phase Alkyldimethylbenzyl-ammoniumchlorid enthält, und die sich zum Abschminken oder zur Hautpflege eignet.

Aus der WO 94/17779 sind Hydrodispersionen bekannt, die ein emulgatorfreies Produkt darstellen, um mögliche Reizwirkungen von Emulgatoren (Mallorca-Akne) zu vermeiden. Die Stabilität der Dispersionen soll durch eine innere Lipidphase mit den anorganischen Pigmenten und eine äußere wäßrige Phase gewährleistet werden.

Der Erfindung liegt die Aufgabe zugrunde, unter Einsatz bekannter Lichtschutzmittel durch besondere Anordnung dieser Mittel in einem Formulierungssystem eine Verbesserung der Lichtschutzeigenschaften herbeizuführen und den direkten Kontakt von chemischen Filtern mit der Haut zu vermeiden.

Erfindungsgemäß besteht das neue Lichtschutzmittel aus wenigstens zwei voneinander getrennt vorliegenden und im wesentlichen flüssigen Phasen, wobei sich UVA- und/oder UVB-Filter und/oder UVC-Filter (im folgenden: UV-Filter) wenigstens in zwei getrennten Phasen befinden, wobei sich die Phasen nach einem kurzzeitigen und schonenden Mischvorgang spontan trennen unter Verbleiben der jeweiligen UV-Filter in der Phase, in der sie ursprünglich enthalten waren.

Es wurde gefunden, daß durch Spreiten auf der Haut von einer der getrennt vorliegenden Phasen und Anordnung wenigstens einer zweiten Phase darüber, wobei sich die zweite mit der ersten Phase im wesentlichen nicht mischt und jede Phase einen UV-Filter enthält, eine verbesserte Absorption der UV-Strahlung gegenüber einer Emulsion oder Hydrodispersion erhalten wird, die die gleiche Menge dieser UV-Filter enthält. Umgesetzt auf den Lichtschutzfaktor bedeutet dies eine Erhöhung um wenigstens zwei Stufen; vorzugsweise 4 bis 10 Stufen.

Es wird jedoch auch schon eine Verbesserung des Lichtschutzfaktors erreicht, wenn nur eine Phase einen UV-Filter enthält, und die andere Phase keinen UV-Filter aber von der einen Phase unterschiedliche Substanzen enthält, da je nach Schichtdicke und gegebenfalls Art der Schicht die Eindringtiefe bzw. Brechung einfallender Strahlung unterschiedlich ist.

Das erfindungsgemäße Lichtschutzmittel, bestehend aus wenigstens zwei getrennten Phasen, vorzugsweise aus drei oder mehr Phasen, kann auf die Haut aufgetragen werden und bildet dort diese Phasen nach dem Auftragen spontan wieder aus. Das bedeutet, der Mischvorgang durch Verreiben des aus einem Vorratsbehälter entnommenen kompletten Lichtschutzmittels bewirkt nur eine momentane Durchmischung, die anschließend durch die Art der gewählten Phasen und deren physikalische Eigenschaften nahezu vollständig wieder aufgehoben wird. Infolge der Phasentrennung ergibt sich ein schichtförmiger Aufbau des Lichtschutzmittels auf der Haut. Dabei sind die UV-Filter so ausgewählt, daß diese nach der Durchmischung wieder in der jeweiligen Phase (Schicht) vorhanden sind, in der sie ursprünglich, d.h. vor dem Mischvorgang enthalten waren.

Ein bevorzugter Aufbau ist beispielsweise folgender, bezogen auf die Hautoberfläche:
(A) eine unmittelbar auf der Haut befindliche, ein oder mehrere Fluorcarbone und als ein UV-Filter ein anorganisches Pigment enthaltende Phase;
(B) eine darüber liegende Phase aus einem polymeren Lack oder einer polymeren filmbildenden Verbindung in einem organischen Lösungsmittel (im folgenden: polymerer Lack); gegebenenfalls im Gemisch mit einem UV-Filter; und
(C) eine darüber liegende ölige Phase mit einem darin enthaltenen UV-Filter.

Dabei beträgt der Anteil der Phasen, bezogen auf die Gesamtzusammensetzung in einer solchen beispielhaften Zusammensetzung etwa
15 bis 35 Gew-% Phase (A); 5 bis 25 Gew-% Phase (B); und 25 bis 80 Gew-% Phase (C).

Es können auch weitere Phasen in das Lichtschutzmittel miteinbezogen werden, z.B eine weitere ölige Phase (D) in Form eines Siliconöles, gegebenenfalls mit einem darin enthaltenen lipophilen UV-Filter, sowie andere Phasen.

Bei einem Schichtaufbau mit drei oder mehr Phasen ist beispielsweise zwischen einer öligen und einer nicht-öligen Schicht eine Phase vorgesehen, die vorzugsweise aus einem polymeren Lack in einem dafür geeigneten hautverträglichen Lösungsmittel besteht. Ein solcher Lack ist zum Beispiel Schellack.

Schellack ist ein natürliches Harz tierischen Ursprungs mit einer durchschnittlichen Molmasse von 1000 g/Mol. Es besteht hauptsächlich aus Hydroxycarbonsäuren, die teilweise ungesättigt sind, Aldehyd-Gruppen enthalten und in Ester - oder Lacton-Form vorliegen. Es verträgt sich gut mit anderen Harzen, Polymeren und Additiven und ist physiologisch und toxikologisch unbedenklich.

Ein geeignetes Polysiloxan-Copolymeres, das in einer öligen Phase enthalten sein kann, ist beispielsweise Poly(dimethylsiloxan) und Poly(isobutylmethacrylat), ein Copolymeres von Poly(dimethylsiloxan) und Polyacrylat, ein Copolymeres von Poly(dimethylsiloxan) und Poly(isobutylmethacrylat)-haltigen Copolymeren (z.B. SA 70-5 von 3M-Company, USA).

Als nicht-ölige Phase eignet sich bevorzugt eine Fluorcarbone, insbesondere Perfluorcarbone enthaltende Phase. Einsetzbare Perfluorcarbone sind Perfluordekalin, Perfluortributylamin, Perfluoroctylbromid, Bis-Fluor(butyl)ethen oder C₆-C₉-Perluoralkane und Gemische dieser untereinander und/oder mit Perfluorpolyethern. Ein bevorzugtes Perfluorcarbon ist Perfluordekalin. Besonders bevorzugt ist ein Gemisch von Perfluordekalin und Perfluorpolymethyl-isopropylether. Darin süspendiert werden UV-Filter, wie z.B. anorganische Oxide oder Melanin. Die Viskosität einer solchen Phase ist über das Molekulargewicht des Perfluorpolyethers leicht einzustellen.

Die Perfluorcarbone selbst sind vollständig untoxisch und gegen äußere Einflüsse wie physikalische und chemische Einflüsse (organische Lösungsmittel, Säuren, Alkali, UV-Bestrahlung, Temperatur) sehr stabil.

Ein vorteilhaftes Mischungsverhältnis der als besonders bevorzugt genannten Mischung liegt beispielsweise im Bereich von 1,5 bis 3 zu 3 bis 1,5.

Ein bevorzugter Phasenaufbau kann darin bestehen, daß diese im wesentlichen kein Wasser enthalten.

Die Phasentrennung ist auf die unterschiedliche Art der phasenbildenden Grundbestandteile zurückzuführen oder auf deutliche Unterschiede in solchen physikalischen Eigenschaften wie z.B. Dichte, Hydrophilie, Hydrophobie. Im allgemeinen wird bei jeder Phase eine ausgeprägte Phasengrenzfläche ausgebildet, die sich vorzugsweise Über die gesamte Phasengrenzfläche der anderen Phase ohne Unterbrechung erstreckt, d.h. eine Bildung von Tröpfchen oder einzelnen Feldern wird vermieden. Die erfindungsgemäß eingesetzten Phasen sind im wesentlichen nicht miteinander mischbar und liegen somit voneinander getrennt vor. Dieser Zustand ist zumindest für den Zeitraum unmittelbar nach der Phasenvermischung bis zu einige Minuten danach stabil, vorzugsweise bis zu einige Stunden danach.

In den jeweiligen Phasen liegt in möglichst homogener Verteilung ein einziger UV-Filter vor. Ein solcher UVB-Filter kann z.B. sein ein Salicylsäureester, wie Salicylsäure-(2-ethylhexyl)ester, Salicylsäurementhylester; Salicylsäure-(4-isopropylbenzyl)ester; ein Benzophenon wie 2-Hydroxy-4-methoxybenzophenon; ein 4-Aminobenzoesäureester, wie 4-(Dimethylamino)-benzoesäure-(2-ethylhexyl)ester, 4-(Dimethylamino)benzoesäureamylester; ein 3-Benzylcampher-Derivat, wie 3-(4-Methylbenzyliden)campher oder 3-Benzylidencampher; ein Zimtsäureester, wie 4-Methoxyzimtsäure(2-ethylhexyl)ester oder 4-Methoxyzimtsäure-isopentylester; oder ein Benzmalonsäureester oder ein Triazin.

Eine Kombination mit UVA-Filtern wie Dibenzoylmenthan-Derivaten, wie 1-(4'-tert-Butylphenyl)-3-(4'-methoxyphenyl)propan-1,3-dion oder 1-Phenyl-3-(4'-isopropylphenol)propan-1,3-dion kann vorteilhaft sein.

Die oben genannten UV-Filter sind lipophil und eignen sich daher zur Einbeziehung in eine oder mehrere der öligen Phasen. Diese Phase kann vorzugsweise zusätzlich auch Radikalfänger wie besipielsweise Vitamin E enthalten.

In die nicht-ölige Phase kann vorteilhaft ein hydrophiler UV-Filter eingebracht werden. Zu hydrophilen UV-Filtern gehören Sulfonsäurederivate von Benzophenonen, wie 2-Hydroxy-4-methoxybenzophenon-5-sulfonsäure und deren Salze; 2-Phenylbenzimidazol-5-sulfonsäure und deren Salze; 3-Benzylidencampher-sulfonsäuren, wie 4-(2-Oxo-3-bornylidenmethyl)benzolsulfonsäure usw. und Salze davon.

Anorganische Pigmente, wie Titaniumdioxid und Zinkoxid und deren Gemische mit Aluminiumoxid und/oder Siliciumdioxid können vorteilhaft in der nicht-öligen Phase als UV-Filter enthalten sein. Dies ist eine besondere erfindungsgemäße Maßnahme, die den Vorteil mit sich bringt, daß bei Anordnung anorganischer Pigmente in der der Haut am nächsten kommenden Phase keine Probleme des Anwenders mit allergischen Reizen durch chemische UV-Filter auftreten. Besonders bevorzugt dabei ist Titaniumdioxid.

Die Teilchengröße der anorganischen Pigmente beträgt dabei vorzugsweise weniger als 400 nm, insbesondere weniger als 300 nm. Auch natürliche Pigmente wie zum Beispiel Melanin können eingesetzt werden.

Im allgemeinen werden in dem Lichtschutzmittel der Erfindung lipophile Filter in der öligen Phase vorhanden sein und hydrophile Filter in der nicht-öligen Phase. Bei einem dreioder mehrschichtigen Aufbau können in einer oder zwei zwischen diesen öligen und nicht-öligen Phasen liegenden weiteren Phasen ebenfalls UV-Filter enthalten sein. Diese Filter sind von den benachbarten Phasen vorzugsweise unterschiedlich, um zu gewährleisten, daß bei der Phasentrennung nach dem Mischvorgang auch der für die Zwischenphase vorgesehene UV-Filter in dieser Schicht wieder enthalten ist. Die Art dieses Filters kann je nach den Filtern in den Nachbarphasen unterschiedlich sein und ist vom Fachmann unter Berücksichtigung der obigen Gesichtspunkte festzulegen.

Es können natürlich auch mehrere UV-Filter in einer Phase enthalten sein, vorausgesetzt in einer zweiten Phase befindet sich wenigsten ein weiterer UV-Filter.

überraschenderweise zeigt sich bei der erfindungsgemäßen Schichtung mehrerer getrennter Phasen übereinander, daß
1. die Absorption von zwei UV-Filtern jeweils allein in einer homogenen Phase (Schicht) wesentlich größer ist, als die Absorption derselben Filter im Gemisch miteinander in nur einer einzigen Phase, gleiche Konzentrationen vorausgesetzt;
2. die Absorption um so höher ist, je homogener der Filter verteilt ist und je dicker die Schicht ist;
3. durch die Anordnung einer Schicht unmittelbar auf der Hautoberfläche allein mit anorganischen Pigmenten (sog. physikalische Filter) eine Beeinträchtigung empfindlicher Haut gegenüber den organischen Filtern (chemische Filter) vermieden wird und dadurch allergische Hautreaktionen ausbleiben;
4. sich auf diese Weise bei schichtartig getrenntem Aufbau eines Lichtschutzmittels gegenüber der üblichen Emulsion ein verringerter Einsatz des Wirkstoffes (UV-Filter) ergibt, oder man erhält bei gleicher Wirkstoffmenge einen höheren Lichtschutzfaktor;
5. ein angenehmes, weiches Hautgefühl (feeling) ohne jegliche Klebrigkeit erhalten wird;
6. hohe Anteile anorganischer Pigmente wie z.B. TiO₂ zu keinem Weißeln-Effekt führen, d.h. die weiße Schlierenbildung bei Wasserkontakt tritt nicht auf.

Ein weiterer Vorteil der vorliegenden Erfindung besteht bei mehrschichtigem Aufbau und Einbeziehung einer Schicht mit einem polymeren Lack darin, daß die Wasserresistenz des Lichtschutzmittels wesentlich verbessert wird. Das ist insbesondere für Lichtschutzmittel ein bedeutender Fortschritt, weil dadurch das oftmalige Auftragen des Mittels auf die Haut beim Baden in Gewässern und Schwimmbädern vermieden wird. Es wurde festgestellt, daß der einmalige Auftrag des Mittels und mehrmaliges Baden danach einen Schutz für wenigstens 3 bis 4 Stunden bedeutet, ohne daß ein nochmaliger Auftrag erfolgen muß.

Die Erfindung betrifft auch ein Verfahren zur Herstellung eines Lichtschutzmittels, das dadurch gekennzeichnet ist, daß wenigstens zwei voneinander getrennt vorliegende und im wesentlichen flüssige Phasen, von denen wenigstens eine, vorzugsweise jedoch zwei Phasen einen UV-Filter enthält/enthalten, in einen Behälter eingebracht werden. Dabei liegen die Phasen in dem Behälter voneinander getrennt oder räumlich voneinander getrennt vor; vorzugsweise liegen die Phasen räumlich voneinander getrennt vor.

Unter "im wesentlichen flüssige Phasen" im Sinne der vorliegenden Erfindung werden Stoffe verstanden, die leichtflüssig bis pasten- oder gel-artig sind und die beim Auftragen auf die Haut noch gleichmäßig verteilbar sind.

Unter "nicht mischbare Phasen" im Sinne der vorliegenden Erfindung werden Stoffe verstanden, die sich mit oder ohne Zugabe irgendwelcher Hilfsmittel entweder nicht oder nur sehr schwer miteinander vermischen bzw. die sich aufeinander ausbreiten, ohne daß eine wesentliche Mischphase entsteht. Der Zustand der Nichtmischbarkeit untereinander sowie der von Flüssigkeiten ist auf den Temperaturbereich von 5 bis 50° C bezogen, normalerweise also Umgebungstemperatur.

"Voneinander getrennt vorliegend" sind Phasen, die eine Schichtbildung zeigen, wenn sie übereinandergeschichtet oder schonend miteinander vermischt werden. "Räumlich voneinander getrennt" sind Phasen, die ohne direkte Berührung miteinander angeordnet sind.

Unter "kurzem und schonenden Mischvorgang" wird ein Mischvorgang verstanden, der weniger als etwa eine Minute beträgt und der ohne wesentliche Krafteinwirkung erfolgt, z.B. durch Ineinanderfließenlassen der Phasen und/oder durch Verreiben der Phasen auf der Haut.

Unter "spontan trennen" wird verstanden, daß nach kurzem und schonendem Mischvorgang eine Schichtausbildung innerhalb von Sekunden bis weniger als 10 Minuten erfolgt.

Das erfindungsgemäße Verfahren zum Auftragen eines Lichtschutzmittels auf die Haut besteht darin, daß in einem Dispenser getrennt vorliegende oder räumlich getrennt vorliegende, im wesentlichen flüssige Phasen, wobei sich UVA- und/oder UVB-Filter wenigstens in zwei getrennten Phasen befinden, unmittelbar vor dem Auftragen in vorbestimmten Mischungsverhältnissen miteinander vermischt und in gemischter Form auf die Haut aufgetragen und verteilt werden.

Ein bevorzugtes Verfahren zum Auftragen des Lichtschutzmittels besteht darin, daß in einem Dispenser räumlich voneinander getrennt vorliegende, im wesentlichen flüssige und wasserfreie Phasen, bestehend aus wenigstens
(1) einer öligen Phase mit einem darin enthaltenen UV-Filter,
(2) einer Phase aus einem polymeren Lack oder einer polymeren filmbildenden Verbindung in einem organischen Lösungsmittel und gegebenenfalls einem UV-Filter enthaltend,
(3) einer ein oder mehrere Fluorcarbone und ein anorganisches Pigment als UV-Filter enthaltenden Phase unmittelbar vor dem Auftragen in vorbestimmten Mischungsverhältnissen kurzzeitig und schonend miteinander vermischt und in gemischter Form auf die Haut aufgetragen und verteilt werden.

Ein Dispenser zum Auftragen des erfindungsgemäßen Lichtschutzmittels kann beispielsweise ein drei Kammern aufweisender und unter Druck stehender oder unter Druck zu setzender Behälter sein, der z.B. in diesen Kammern in vorbestimmter Menge die Einzelphasen enthält. Diese werden dann durch Betätigung des Druckventils in einem Mischkopf im gewünschten Mischungsverhältnis miteinander vermischt und durch eine Ausgabeöffnung herausgedrückt. Im Anschluß daran erfolgt die Verteilung auf der Haut durch den Anwender.

Die Erfindung soll nachstehend durch Beispiele näher erläutert werden. Alle Prozentangaben sind auf das Gewicht (die Masse) bezogen, sofern keine anderslautenden Angaben gemacht wurden.

Die Herstellung der einzelnen Phasen erfolgt in der Weise, daß für die Phase A das anorganische Pigment, z.B TiO₂, und gegebenenfalls ein Farbpigment im flüssigen Medium dispergiert werden. Bei Phase B werden der polymere Lack oder Filmbildner und gegebenenfalls chemische UV-Filter in das Lösungsmittel gegeben und gemischt. Für die Phase C oder weitere Phasen werden in die flüssigen öle oder Siliconpolymeren chemische Filter, gegebenenfalls auch in Verbindung mit Antioxidantien wie Vitamin E, eingetragen und miteinander vermischt.

### Beispiel 1

Es wurden die einzelnen Phasen getrennt hergestellt durch Vermischen der jeweiligen Bestandteile in oben angegebener Weise.

| Phase A | |
|---|---|
| Perfluordekalin | 60 % |
| Perfluorpolymethylisopropylether (Fomblin HC) | 30 % |
| UV-Filter TiO₂ | 10 % |

| Phase B | |
|---|---|
| Schellack | 1,0 % |
| UV-Filter Benzophenon-3 (2-Hydroxy-4-methoxybenzophenon) | 2,0 % |
| UV-Filter Octylmethoxycinnamat (2-Ethylhexyl-p-methoxycinnamat) | 4,5 % |
| Isopropanol | q.s. |

| Phase C | |
|---|---|
| Jojobaöl | 5,0 % |
| UV-Filter Benzophenon-3 | 6,0 % |
| Vitamin E | 1,0 % |
| UV-Filter Octylmethoxycinnamat | 5,5 % |
| Dioctylether | q.s. |
| Der Anteil der Phasen A, B bzw. C betrug im Gesamtgemisch 35 % , 20 % bzw. 45 %. | |

### Beispiel 2

Es wurden die einzelnen Phasen getrennt hergestellt durch Vermischen der jeweiligen Bestandteile in fachgemäßer Weise.

| Phase A | |
|---|---|
| Perfluordekalin | q.s |
| UV-Filter TiO₂ | 8 % |
| Zinkoxid | 7 % |
| Kaolin/siliciumdioxid | 10 % |

| Phase B | |
|---|---|
| Schellack | 3,0 % |
| UV-Filter Natrium- und Triethanolaminsalze von 2-Phenylbenzimidazol-5-sulfonsäure | 20,0 % |
| UV-Filter Octylmethoxycinnamat | 4,5 % |
| Ethanol | q.s. |
| | |

| Phase C | |
|---|---|
| Jojobaöl | 7,5 % |
| Isohexadecan | 3,5 % |
| Vitamin E | 1,0 % |
| UV-Filter octylmethoxycinnamat | 15,5 % |
| Oxybenzon | 9,5 % |
| Dioctylether | q.s. |
| Der Anteil der Phasen A, B bzw. C betrug im Gesamtgemisch 15 % , 5 % bzw. 80 %. | |

### Beispiel 3

Es wurden die einzelnen Phasen getrennt hergestellt durch Vermischen der jeweiligen Bestandteile in fachgemäßer Weise.

| Phase A | |
|---|---|
| C₁₂-C₁₅-Alkylbenzoat | q.s. |
| UV-Filter TiO₂ | 20 % |

| Phase B | |
|---|---|
| Isopropanol | q.s |
| UV-Filter Natrium- und Triethanolaminsalze von 2-Phenylbenzimidazol-5-sulfonsäure | 10,0 % |

| Phase C | |
|---|---|
| Siliconöl | q.s |
| Poly(dimethylsiloxan)-g-poly(isobutylmethacrylat) | 25 % |
| UV-Filter Oxybenzon | 10 % |
| UV-Filter Octylmethoxycinnamat | 5 % |
| Der Anteil der Phasen A, B bzw. C betrug im Gesamtgemisch 30 % , 20 % bzw. 50 %. | |

### Beispiel 4 (Sonnenschutz als leichtes Make up)

Es wurden die einzelnen Phasen getrennt hergestellt durch Vermischen der jeweiligen Bestandteile in fachgemäßer Weise.

| Phase A | |
|---|---|
| C₁₂-C₁₅-Alkylbenzoat | q.s. |
| UV-Filter TiO₂ | 5 % |
| Zinkoxid | 2,5 % |
| Kaolin* | 5,0 % |
| pigmentierte Farben für Make up | 2,0 % |
| lösliche Farben | 1,5 % |

| Phase B | |
|---|---|
| Schellack | 0,5 % |
| Isopropanol | q.s |
| UV-Filter Natrium- und Triethanolaminsalze von 2-Phenylbenzimidazol-5-sulfonsäure | 15,0 % |

| Phase C | |
|---|---|
| Siliconöl | q.s |
| Poly(isobutylmethacrylat)-co-methyl FOSEA)-g-Poly(dimethylsiloxan) | 20 % |
| UV-Filter Oxybenzon | 8 % |

| | |
|---|---|
| * Kaolin mit hohem Kaolinitgehalt und 0,5 - 10 Gew.-% sphärischem SiO₂, das eine Teilchengröße < 5µm hat (gemäß Patentanmeldung P 44 45 064.8). | |

Der Anteil der Phasen A, B bzw. C betrug im Gesamtgemisch 45 % , 10 % bzw. 45 %.

### Beispiel 5 (leichter Selbstbräuner mit hohem Sonnenschutz)

| Liposome mit DHA | |
|---|---|
| Lecithin | 10 % |
| Dihydroxyaceton (DHA) | 10 % |
| Ethanol | 7 % |
| Wasser | q.s |

Das Dihydroxyaceton wird in Wasser gelöst und in Lecithin eingerührt. Danach wird Ethanol eingerührt und das Ganze gut homogenisiert.

| Phase A | |
|---|---|
| Perfluordekalin | q.s |
| TiO₂ | 8 % |

| Phase B | |
|---|---|
| Isopropanol | q.s |
| Schellack | 1,5 % |
| UV-Filter Natrium- und Triethanolaminsalze von 2-Phenylbenzimidazol-5-sulfonsäure | 10,0 % |

| Phase C | |
|---|---|
| PPG-isostearylether | q.s |
| UV-Filter Butylmethoxydibenzoylmenthan | 5,0 % |
| 4-Methoxybenzylidencampher | 8,0 % |
| Dihydroxyaceton-Liposome | 10,0 % |

### Beispiel 6

| Phase A | |
|---|---|
| Perfluordekalin | q.s |
| TiO₂ | 8 % |
| Melanin | 1 % |

| Phase B | |
|---|---|
| Ethanol | q.s |
| Schellack | 1,5 % |
| Natrium- und Triethanolaminsalze von 2-Phenylbenzimidazol-5-sulfonsäure | 15% |

| Phase C | |
|---|---|
| Jojobaöl | q.s |
| Isohexadecan | 3 % |
| Oxybenzon | 10 % |
| Octylmethoxycinnamat | 15 % |
| Melanin löslich | 3 % |
| Der Anteil der Phasen A, B bzw. C betrug im Gesamtgemisch 35 % , 15 % bzw. 50 %. | |

### Beispiel 7 (Make up mit Selbstbräuner und hohem Lichtschutz)

| Phase A | |
|---|---|
| C₁₂-C₁₅-Alkylbenzoat | q.s. |
| UV-Filter TiO₂ | 5 % |
| Zinkoxid | 1,5 % |
| Kaolin* | 4,5 % |
| pigmentierte Farben für Make up | 2,0 % |
| lösliche Farben je nach Farbton | 1,0 % |
| Melanin | 1,0 % |
| DHA-Liposome gemäß Beispiel 5 | 10 % |

| Phase B | |
|---|---|
| Schellack | 1,0 % |
| Isopropanol | q.s |
| Natrium- und Triethanolaminsalze von 2-Phenylbenzimidazol-5-sulfonsäure | 15 % |

| Phase C | |
|---|---|
| Siliconöl | q.s. |
| Poly(isobutylmethacrylat)-co-methyl FOSEA)-g-Poly(dimethylsiloxan) | 20 % |
| Oxybenzon | 8 % |
| Vitamin E | 1 % |
| Melanin löslich | 1,5 % |

### Beispiel 8

Es wurden 3 mg einer öligen Phase (auf Basis eines Linolates) pro cm² Haut bei 10 Probanden aufgetragen. Diese Phase enthielt 2 % Octylmethoxycinnamat (OMC) als UV-Filter. Darüber wurden 1 mg/cm² einer wäßrigen Phase aufgetragen. Die wäßrige Phase enthielt 3 % TiO₂ , so daß 4 mg/cm² insgesamt 0,14 mg OMC/cm² und 0,2 mg TiOVEIL AQN/cm² aufwiesen. Nach Messung des Lichtschutzfaktors nach dem Diffey-Verfahren ergab sich ein Durchschnittswert von LSF = 7,03.

### Vergleichsbeispiel 1

Es wurde eine Vergleichsemulsion mit 4 mg/cm² Haut bei 10 Probanden aufgetragen. Die Emulsion enthielt 1 % Titaniumdioxid-Filter und 1,5 % des OMC-Filters. Diese Vergleichsemulsion enthielt somit die gleiche Menge UV-Filter wie die beiden Schichten in Beispiel 8 gemeinsam. Nach Messung des Lichtschutzfaktors nach dem Diffey-Verfahren ergab sich ein Durchschnittswert von 4,97.

Der Vergleich zeigt deutlich, daß bereits bei zweischichtiger Anordnung von Lichtschutzmitteln wesentliche Verbesserungen gegenüber den bekannten Emulsionen erreicht werden können.

Die Erfindung betrifft ein neues mehrphasiges Lichtschutzmittel mit verbesserten Lichtschutzeigenschaften.

Der Anmelder hat sich unter Bezugnahme auf die DE 44 26 952 C2 freiwillig eingeschränkt und gesonderte Patentansprüche für DE vorgelegt.

Es sind bereits eine Vielzahl von Lichtschutzformulierungen bekannt, die als Emulsionen für die Ausfilterung (Absorption oder Reflexion) der UVB-Strahlung (Bereich 290 bis 320 nm) und der UVA-Strahlung (Bereich 320 bis 400 nm) bestimmte organische und anorganische Wirkstoffe enthalten. Als organische Wirkstoffe sind insbesondere die Derivate des Dibenzoylmethans, des Benzophenons, der Salicylsäure, der Zimtsäure, des 3-Benzylidencamphers, der 4-Aminobenzoesäure usw. bekannt. Als anorganische Wirkstoffe sind Pigmente bekannt, wie die Oxide von Titanium, Eisen, Zink, Silicium, Aluminium, Zirkonium usw. Ein dem Titaniumdioxid als überlegen bezeichnetes Gemisch von organischen und anorganischen Stoffen ist ebenfalls bekannt (EP-A-588498).

In der Hauptsache ist die Entwicklung bei den Lichtschutzformulierungen zu neuen Wirkstoffen hingegangen bzw. zur Kombination von bekannten Wirkstoffen. Allgemein liegen diese in Emulsionen vor, um eine gute und gleichmäßige Verteilung auf der Haut zu gewährleisten, wobei Agglomerationen von anorganischen Pigmenten durch grenzflächenaktive Stoffe verhindert wurden.

Es sind jedoch auch Hydrodispersionen bekannt geworden, bei denen ein Emulgator nicht vorhanden ist, und die eine Dispersion einer diskontinuierlichen Lipidphase (flüssig, fest oder halbfest) in einer äußeren kontinuierlichen (wäßrigen) Phase darstellen. Die Stabilität eines solchen Emulgator-freien Systems wird z.B. durch Aufbau eines Gelgerüstes in der wäßrigen Phase erreicht mit einer stabilen Suspendierung der Lipidtröpfchen in diesem Gelgerüst. Nachteilig bei diesen Hydrodispersionen ist die notwendige hohe Konzentration von UV-Filtermaterialien und deren Klebrigkeit.

Die EP-A-603080 beschreibt eine kosmetische oder dermatologische Zwei-Phasen-Zusammensetzung, bestehend aus einer wäßrigen und einer separaten ölphase, die in der wäßrigen Phase Alkyldimethylbenzyl-ammoniumchlorid enthält, und die sich zum Abschminken oder zur Hautpflege eignet.

Aus der WO 94/17779 sind Hydrodispersionen bekannt, die ein emulgatorfreies Produkt darstellen, um mögliche Reizwirkungen von Emulgatoren (Mallorca-Akne) zu vermeiden. Die Stabilität der Dispersionen soll durch eine innere Lipidphase mit den anorganischen Pigmenten und eine äußere wäßrige Phase gewährleistet werden.

Der Erfindung liegt die Aufgabe zugrunde, unter Einsatz bekannter Lichtschutzmittel durch besondere Anordnung dieser Mittel in einem Formulierungssystem eine Verbesserung der Lichtschutzeigenschaften herbeizuführen und den direkten Kontakt von chemischen Filtern mit der Haut zu vermeiden.

Erfindungsgemäß besteht das neue Lichtschutzmittel aus wenigstens zwei voneinander getrennt vorliegenden und im wesentlichen flüssigen Phasen, wobei sich UVA- und/oder UVB-Filter und/oder UVC-Filter (im folgenden: UV-Filter) wenigstens in zwei getrennten Phasen befinden, wobei sich die Phasen nach einem kurzzeitigen und schonenden Mischvorgang spontan trennen unter Verbleiben der jeweiligen UV-Filter in der Phase, in der sie ursprünglich enthalten waren.

Es wurde gefunden, daß durch Spreiten auf der Haut von einer der getrennt vorliegenden Phasen und Anordnung wenigstens einer zweiten Phase darüber, wobei sich die zweite mit der ersten Phase im wesentlichen nicht mischt und jede Phase einen UV-Filter enthält, eine verbesserte Absorption der UV-Strahlung gegenüber einer Emulsion oder Hydrodispersion erhalten wird, die die gleiche Menge dieser UV-Filter enthält. Umgesetzt auf den Lichtschutzfaktor bedeutet dies eine Erhöhung um wenigstens zwei Stufen, vorzugsweise 4 bis 10 Stufen.

Es wird jedoch auch schon eine Verbesserung des Lichtschutzfaktors erreicht, wenn nur eine Phase einen UV-Filter enthält, und die andere Phase keinen UV-Filter aber von der einen Phase unterschiedliche Substanzen enthält, da je nach Schichtdicke und gegebenfalls Art der Schicht die Eindringtiefe bzw. Brechung einfallender Strahlung unterschiedlich ist.

Das erfindungsgemäße Lichtschutzmittel, bestehend aus wenigstens zwei getrennten Phasen, vorzugsweise aus drei oder mehr Phasen, kann auf die Haut aufgetragen werden und bildet dort diese Phasen nach dem Auftragen spontan wieder aus. Das bedeutet, der Mischvorgang durch Verreiben des aus einem Vorratsbehälter entnommenen kompletten Lichtschutzmittels bewirkt nur eine momentane Durchmischung, die anschließend durch die Art der gewählten Phasen und deren physikalische Eigenschaften nahezu vollständig wieder aufgehoben wird. Infolge der Phasentrennung ergibt sich ein schichtförmiger Aufbau des Lichtschutzmittels auf der Haut. Dabei sind die UV-Filter so ausgewählt, daß diese nach der Durchmischung wieder in der jeweiligen Phase (Schicht) vorhanden sind, in der sie ursprünglich, d.h. vor dem Mischvorgang enthalten waren.

Ein bevorzugter Aufbau ist beispielsweise folgender, bezogen auf die Hautoberfläche:
(A) eine unmittelbar auf der Haut befindliche, ein oder mehrere Fluorcarbone und als ein UV-Filter ein anorganisches Pigment enthaltende Phase;
(B) eine darüber liegende Phase aus einem polymeren Lack oder einer polymeren filmbildenden Verbindung in einem organischen Lösungsmittel (im folgenden: polymerer Lack), gegebenenfalls im Gemisch mit einem UV-Filter; und
(C) eine darüber liegende ölige Phase mit einem darin enthaltenen UV-Filter.

Dabei beträgt der Anteil der Phasen, bezogen auf die Gesamtzusammensetzung in einer solchen beispielhaften Zusammensetzung etwa
15 bis 35 Gew-% Phase (A); 5 bis 25 Gew-% Phase (B); und 25 bis 80 Gew-% Phase (C).

Es können auch weitere Phasen in das Lichtschutzmittel miteinbezogen werden, z.B eine weitere ölige Phase (D) in Form eines Siliconöles, gegebenenfalls mit einem darin enthaltenen lipophilen UV-Filter, sowie andere Phasen.

Bei einem Schichtaufbau mit drei oder mehr Phasen ist beispielsweise zwischen einer öligen und einer nicht-öligen Schicht eine Phase vorgesehen, die vorzugsweise aus einem polymeren Lack in einem dafür geeigneten hautverträglichen Lösungsmittel besteht. Ein solcher Lack ist zum Beispiel Schellack.

Schellack ist ein natürliches Harz tierischen Ursprungs mit einer durchschnittlichen Molmasse von 1000 g/Mol. Es besteht hauptsächlich aus Hydroxycarbonsäuren, die teilweise ungesättigt sind, Aldehyd-Gruppen enthalten und in Ester - oder Lacton-Form vorliegen. Es verträgt sich gut mit anderen Harzen, Polymeren und Additiven und ist physiologisch und toxikologisch unbedenklich.

Ein geeignetes Polysiloxan-Copolymeres, das in einer öligen Phase enthalten sein kann, ist beispielsweise Poly(dimethylsiloxan) und Poly(isobutylmethacrylat), ein Copolymeres von Poly(dimethylsiloxan) und Polyacrylat, ein Copolymeres von Poly(dimethylsiloxan) und Poly(isobutylmethacrylat)-haltigen Copolymeren (z.B. SA 70-5 von 3M-Company, USA).

Als nicht-ölige Phase eignet sich bevorzugt eine Fluorcarbone, insbesondere Perfluorcarbone enthaltende Phase. Einsetzbare Perfluorcarbone sind Perfluordekalin, Perfluortributylamin, Perfluoroctylbromid, Bis-Fluor(butyl)ethen oder C₆-C₉-Perluoralkane und Gemische dieser untereinander und/oder mit Perfluorpolyethern. Ein bevorzugtes Perfluorcarbon ist Perfluordekalin. Besonders bevorzugt ist ein Gemisch von Perfluordekalin und Perfluorpolymethyl-isopropylether. Darin suspendiert werden UV-Filter, wie z.B. anorganische Oxide oder Melanin. Die Viskosität einer solchen Phase ist über das Molekulargewicht des Perfluorpolyethers leicht einzustellen.

Die Perfluorcarbone selbst sind vollständig untoxisch und gegen äußere Einflüsse wie physikalische und chemische Einflüsse (organische Lösungsmittel, Säuren, Alkali, UV-Bestrahlung, Temperatur) sehr stabil.

Ein vorteilhaftes Mischungsverhältnis der als besonders bevorzugt genannten Mischung liegt beispielsweise im Bereich von 1,5 bis 3 zu 3 bis 1,5.

Ein bevorzugter Phasenaufbau kann darin bestehen, daß diese im wesentlichen kein Wasser enthalten.

Die Phasentrennung ist auf die unterschiedliche Art der phasenbildenden Grundbestandteile zurückzuführen oder auf deutliche Unterschiede in solchen physikalischen Eigenschaften wie Z.B. Dichte, Hydrophilie, Hydrophobie. Im allgemeinen wird bei jeder Phase eine ausgeprägte Phasengrenzfläche ausgebildet, die sich vorzugsweise über die gesamte Phasengrenzfläche der anderen Phase ohne Unterbrechung erstreckt, d.h. eine Bildung von Tröpfchen oder einzelnen Feldern wird vermieden. Die erfindungsgemäß eingesetzten Phasen sind im wesentlichen nicht miteinander mischbar und liegen somit voneinander getrennt vor. Dieser Zustand ist zumindest für den Zeitraum unmittelbar nach der Phasenvermischung bis zu einige Minuten danach stabil, vorzugsweise bis zu eihige Stunden danach.

In den jeweiligen Phasen liegt in möglichst homogener Verteilung ein einziger UV-Filter vor. Ein solcher UVB-Filter kann z.B. sein ein Salicylsäureester, wie Salicylsäüre-(2-ethylhexyl)ester, Salicylsäurementhylester, Salicylsäure-(4-isopropylbenzyl)ester; ein Benzophenon wie 2-Hydroxy-4-methoxybenzophenon; ein 4-Aminobenzoesäureester, wie 4-(Dimethyl-amino)-benzoesäure-(2-ethylhexyl)ester, 4-(Dimethylamino)benzoesäureamylester; ein 3-Benzylcampher-Derivat, wie 3-(4-Methylbenzyliden)campher oder 3-Benzylidencampher; ein Zimtsäureester, wie 4-Methoxyzimtsäure(2-ethylhexyl)ester oder 4-Methoxyzimtsäure-isopentylester; oder ein Benzmalonsäureester oder ein Triazin.

Eine Kombination mit UVA-Filtern wie Dibenzoylmenthan-Derivaten, wie 1-(4'-tert-Butylphenyl)-3-(4'-methoxyphenyl)propan-1,3-dion oder 1-Phenyl-3-(4'-isopropylphenol)propan-1,3-dion kann vorteilhaft sein.

Die oben genannten UV-Filter sind lipophil und eignen sich daher zur Einbeziehung in eine oder mehrere der öligen Phasen. Diese Phase kann vorzugsweise zusätzlich auch Radikalfänger wie besipielsweise Vitamin E enthalten.

In die nicht-ölige Phase kann vorteilhaft ein hydrophiler UV-Filter eingebracht werden. Zu hydrophilen UV-Filtern gehören
Sulfonsäurederivate von Benzophenonen, wie 2-Hydroxy-4-methoxybenzophenon-5-sulfonsäure und deren Salze;
2-Phenylbenzimidazol-5-sulfonsäure und deren Salze;
3-Benzylidencampher-sulfonsäuren, wie 4-(2-Oxo-3-bornylidenmethyl)benzolsulfonsäure usw. und Salze davon.

Anorganische Pigmente, wie Titaniumdioxid und Zinkoxid und deren Gemische mit Aluminiumoxid und/oder Siliciumdioxid können vorteilhaft in der nicht-öligen Phase als UV-Filter enthalten sein. Dies ist eine besondere erfindungsgemäße Maßnahme, die den Vorteil mit sich bringt, daß bei Anordnung anorganischer Pigmente in der der Haut am nächsten kommenden Phase keine Probleme des Anwenders mit allergischen Reizen durch chemische UV-Filter auftreten. Besonders bevorzugt dabei ist Titaniumdioxid.

Die Teilchengröße der anorganischen Pigmente beträgt dabei vorzugsweise weniger als 400 nm, insbesondere weniger als 300 nm. Auch natürliche Pigmente wie zum Beispiel Melanin können eingesetzt werden.

Im allgemeinen werden in dem Lichtschutzmittel der Erfindung lipophile Filter in der öligen Phase vorhanden sein und hydrophile Filter in der nicht-öligen Phase. Bei einem dreioder mehrschichtigen Aufbau können in einer oder zwei zwischen diesen öligen und nicht-öligen Phasen liegenden weiteren Phasen ebenfalls UV-Filter enthalten sein. Diese Filter sind von den benachbarten Phasen vorzugsweise unterschiedlich, um zu gewährleisten, daß bei der Phasentrennung nach dem Mischvorgang auch der für die Zwischenphase vorgesehene UV-Filter in dieser Schicht wieder enthalten ist. Die Art dieses Filters kann je nach den Filtern in den Nachbarphasen unterschiedlich sein und ist vom Fachmann unter Berücksichtigung der obigen Gesichtspunkte festzulegen.

Es können natürlich auch mehrere UV-Filter in einer Phase enthalten sein, vorausgesetzt in einer zweiten Phase befindet sich wenigsten ein weiterer UV-Filter.

Überraschenderweise zeigt sich bei der erfindungsgemäßen Schichtung mehrerer getrennter Phasen übereinander, daß
1. die Absorption von zwei UV-Filtern jeweils allein in einer homogenen Phase (Schicht) wesentlich größer ist, als die Absorption derselben Filter im Gemisch miteinander in nur einer einzigen Phase, gleiche Konzentrationen vorausgesetzt;
2. die Absorption um so höher ist, je homogener der Filter verteilt ist und je dicker die Schicht ist;
3. durch die Anordnung einer Schicht unmittelbar auf der Hautoberfläche allein mit anorganischen Pigmenten (sog. physikalische Filter) eine Beeinträchtigung empfindlicher Haut gegenüber den organischen Filtern (chemische Filter) vermieden wird und dadurch allergische Hautreaktionen ausbleiben;
4. sich auf diese Weise bei schichtartig getrenntem Aufbau eines Lichtschutzmittels gegenüber der üblichen Emulsion ein verringerter Einsatz des Wirkstoffes (UV-Filter) ergibt, oder man erhält bei gleicher Wirkstoffmenge einen höheren Lichtschutzfaktor;
5. ein angenehmes, weiches Hautgefühl (feeling) ohne jegliche Klebrigkeit erhalten wird;
6. hohe Anteile anorganischer Pigmente wie z.B. TiO₂ zu keinem Weißeln-Effekt führen, d.h. die weiße Schlierenbildung bei Wasserkontakt tritt nicht auf.

Ein weiterer Vorteil der vorliegenden Erfindung besteht bei mehrschichtigem Aufbau und Einbeziehung einer Schicht mit einem polymeren Lack darin, daß die Wasserresistenz des Lichtschutzmittels wesentlich verbessert wird. Das ist insbesondere für Lichtschutzmittel ein bedeutender Fortschritt, weil dadurch das oftmalige Auftragen des Mittels auf die Haut beim Baden in Gewässern und Schwimmbädern vermieden wird. Es wurde festgestellt, daß der einmalige Auftrag des Mittels und mehrmaliges Baden danach einen Schutz für wenigstens 3 bis 4 Stunden bedeutet, ohne daß ein nochmaliger Auftrag erfolgen muß.

Die Erfindung betrifft auch ein Verfahren zur Herstellung eines Lichtschutzmittels, das dadurch gekennzeichnet ist, daß wenigstens zwei voneinander getrennt vorliegende und im wesentlichen flüssige Phasen, von denen wenigstens eine, vorzugsweise jedoch zwei Phasen einen UV-Filter enthält/enthalten, in einen Behälter eingebracht werden. Dabei liegen die Phasen in dem Behälter voneinander getrennt oder räumlich voneinander getrennt vor; vorzugsweise liegen die Phasen räumlich voneinander getrennt vor.

Unter "im wesentlichen flüssige Phasen" im Sinne der vorliegenden Erfindung werden Stoffe verstanden, die leichtflüssig bis pasten- oder gel-artig sind und die beim Auftragen auf die Haut noch gleichmäßig verteilbar sind.

Unter "nicht mischbare Phasen" im Sinne der vorliegenden Erfindung werden Stoffe verstanden, die sich mit oder ohne Zugabe irgendwelcher Hilfsmittel entweder nicht oder nur sehr schwer miteinander vermischen bzw. die sich aufeinander ausbreiten, ohne daß eine wesentliche Mischphase entsteht. Der Zustand der Nichtmischbarkeit untereinander sowie der von Flüssigkeiten ist auf den Temperaturbereich von 5 bis 50° C bezogen, normalerweise also Umgebungstemperatur.

"Voneinander getrennt vorliegend" sind Phasen, die eine Schichtbildung zeigen, wenn sie übereinandergeschichtet oder schonend miteinander vermischt werden. "Räumlich voneinander getrennt" sind Phasen, die ohne direkte Berührung miteinander angeordnet sind.

Unter "kurzem und schonenden Mischvorgang" wird ein Mischvorgang verstanden, der weniger als etwa eine Minute beträgt und der ohne wesentliche Krafteinwirkung erfolgt, z.B. durch Ineinanderfließenlassen der Phasen und/oder durch Verreiben der Phasen auf der Haut.

Unter "spontan trennen" wird verstanden, daß nach kurzem und schonendem Mischvorgang eine Schichtausbildung innerhalb von Sekunden bis weniger als 10 Minuten erfolgt.

Das erfindungsgemäße Verfahren zum Auftragen eines Lichtschutzmittels auf die Haut besteht darin, daß in einem Dispenser getrennt vorliegende oder räumlich getrennt vorliegende, im wesentlichen flüssige Phasen, wobei sich UVA- und/oder UVB-Filter wenigstens in zwei getrennten Phasen befinden, unmittelbar vor dem Auftragen in vorbestimmten Mischungsverhältnissen miteinander vermischt und in gemischter Form auf die Haut aufgetragen und verteilt werden.

Ein bevorzugtes Verfahren zum Auftragen des Lichtschutzmittels besteht darin, daß in einem Dispenser räumlich voneinander getrennt vorliegende, im wesentlichen flüssige und wasserfreie Phasen, bestehend aus wenigstens
(1) einer öligen Phase mit einem darin enthaltenen UV-Filter,
(2) einer Phase aus einem polymeren Lack oder einer polymeren filmbildenden Verbindung in einem organischen Lösungsmittel und gegebenenfalls einem UV-Filter enthaltend,
(3) einer ein oder mehrere Fluorcarbone und ein anorganisches Pigment als UV-Filter enthaltenden Phase
   unmittelbar vor dem Auftragen in vorbestimmten Mischungsverhältnissen kurzzeitig und schonend miteinander vermischt und in gemischter Form auf die Haut aufgetragen und verteilt werden.

Ein Dispenser zum Auftragen des erfindungsgemäßen Lichtschutzmittels kann beispielsweise ein drei Kammern aufweisender und unter Druck stehender oder unter Druck zu setzender Behälter sein, der z.B. in diesen Kammern in vorbestimmter Menge die Einzelphasen enthält. Diese werden dann durch Betätigung des Druckventils in einem Mischkopf im gewünschten Mischungsverhältnis miteinander vermischt und durch eine Ausgabeöffnung herausgedrückt. Im Anschluß daran erfolgt die Verteilung auf der Haut durch den Anwender.

Die Erfindung soll nachstehend durch Beispiele näher erläutert werden. Alle Prozentangaben sind auf das Gewicht (die Masse) bezogen, sofern keine anderslautenden Angaben gemacht wurden.

Die Herstellung der einzelnen Phasen erfolgt in der Weise, daß für die Phase A das anorganische Pigment, z.B TiO₂, und gegebenenfalls ein Farbpigment im flüssigen Medium dispergiert werden. Bei Phase B werden der polymere Lack oder Filmbildner und gegebenenfalls chemische UV-Filter in das Lösungsmittel gegeben und gemischt. Für die Phase C oder weitere Phasen werden in die flüssigen öle oder Siliconpolymeren chemische Filter, gegebenenfalls auch in Verbindung mit Antioxidantien wie Vitamin E, eingetragen und miteinander vermischt.

### Beispiel 1

Es wurden die einzelnen Phasen getrennt hergestellt durch Vermischen der jeweiligen Bestandteile in oben angegebener Weise.

| Phase A | |
|---|---|
| Perfluordekalin | 60 % |
| Perfluorpolymethylisopropylether | 30 % |
| (Fomblin HC) | |
| UV-Filter TiO₂ | 10 % |

| Phase B | |
|---|---|
| Schellack | 1,0 % |
| UV-Filter Benzophenon-3 | |
| (2-Hydroxy-4-methoxybenzophenon) | 2,0 % |
| UV-Filter Octylmethoxycinnamat (2-Ethylhexyl-p-methoxycinnamat) | 4,5 % |
| Isopropanol | q.s. |

| Phase C | |
|---|---|
| Jojobaöl | 5,0 % |
| UV-Filter Benzophenon-3 | 6,0 % |
| Vitamin E | 1,0 % |
| UV-Filter Octylmethoxycinnamat | 5,5 % |
| Dioctylether | q.s. |
| Der Anteil der Phasen A, B bzw. C betrug im Gesamtgemisch 35 % , 20 % bzw. 45 %. | |

### Beispiel 2

Es wurden die einzelnen Phasen getrennt hergestellt durch Vermischen der jeweiligen Bestandteile in fachgemäßer Weise.

| Phase A | |
|---|---|
| Perfluordekalin | q.s |
| UV-Filter TiO₂ | 8 % |
| Zinkoxid | 7 % |
| Kaolin/Siliciumdioxid | 10 % |

| Phase B | |
|---|---|
| Schellack | 3,0 % |
| UV-Filter Natrium- und Triethanolaminsalze von 2-Phenylbenzimidazol-5-sulfonsäure | 20,0 % |
| UV-Filter Octylmethoxycinnamat | 4,5 % |
| Ethanol | q.s. |
| | |

| Phase C | |
|---|---|
| Jojobaöl | 7,5 % |
| Isohexadecan | 3,5 % |
| Vitamin E | 1,0 % |
| UV-Filter Octylmethoxycinnamat | 15,5 % |
| Oxybenzon | 9,5 % |
| Dioctylether | q.s. |
| Der Anteil der Phasen A, B bzw. C betrug im Gesamtgemisch 15 % , 5 % bzw. 80 %. | |

### Beispiel 3

Es wurden die einzelnen Phasen getrennt hergestellt durch Vermischen der jeweiligen Bestandteile in fachgemäßer Weise.

| Phase A | |
|---|---|
| C₁₂-C₁₅-Alkylbenzoat | q.s. |
| UV-Filter TiO₂ | 20 % |

| Phase B | |
|---|---|
| Isopropanol | q.s |
| UV-Filter Natrium- und Triethanolaminsalze von 2-Phenylbenzimidazol-5-sulfonsäure | 10,0 % |

| Phase C | |
|---|---|
| Siliconöl | q.s |
| Poly(dimethylsiloxan)-g-poly(isobutylmethacrylat) | 25 % |
| UV-Filter Oxybenzon | 10 % |
| UV-Filter Octylmethoxycinnamat | 5 % |
| Der Anteil der Phasen A; B bzw. C betrug im Gesamtgemisch 30 % , 20 % bzw. 50 %. | |

### Beispiel 4 (Sonnenschutz als leichtes Make up)

Es wurden die einzelnen Phasen getrennt hergestellt durch Vermischen der jeweiligen Bestandteile in fachgemäßer Weise.

| Phase A | |
|---|---|
| C₁₂-C₁₅-Alkylbenzoat | q.s. |
| UV-Filter TiO₂ | 5 % |
| Zinkoxid | 2,5 % |
| Kaolin* | 5,0 % |
| pigmentierte Farben für Make up | 2,0 % |
| lösliche Farben | 1,5 % |

| Phase B | |
|---|---|
| Schellack | 0,5 % |
| Isopropanol | q.s |
| UV-Filter Natrium- und Triethanolaminsalze von 2-Phenylbenzimidazol-5-sulfonsäure | 15,0 % |

| Phase C | |
|---|---|
| Siliconöl | q.s |
| Poly(isobutylmethacrylat)-co-methyl FOSEA)-g-Poly(dimethylsiloxan) | 20 % |
| UV-Filter Oxybenzon | 8 % |

| | |
|---|---|
| * Kaolin mit hohem Kaolinitgehalt und 0,5 - 10 Gew.-% sphärischem SiO₂, das eine Teilchengröße < 5µm hat (gemäß Patentanmeldung P 44 45 064.8). | |

Der Anteil der Phasen A, B bzw. C betrug im Gesamtgemisch 45 % , 10 % bzw. 45 %.

### Beispiel 5 (leichter Selbstbräuner mit hohem Sonnenschutz)

| Liposome mit DHA | |
|---|---|
| Lecithin | 10 % |
| Dihydroxyaceton (DHA) | 10 % |
| Ethanol | 7 % |
| Wasser | q.s |

Das Dihydroxyaceton wird in Wasser gelöst und in Lecithin eingerührt. Danach wird Ethanol eingerührt und das Ganze gut homogenisiert.

| Phase A | |
|---|---|
| Perfluordekalin | q.s |
| TiO₂ | 8 % |

| Phase B | |
|---|---|
| Isopropanol | q.s |
| Schellack | 1,5 % |
| UV-Filter Natrium- und Triethanolaminsalze von 2-Phenylbenzimidazol-5-sulfonsäure | 10,0 % |

| Phase C | |
|---|---|
| PPG-isostearylether | q.s |
| UV-Filter Butylmethoxydibenzoylmenthan | 5,0 % |
| 4-Methoxybenzylidencampher | 8,0 % |
| Dihydroxyaceton-Liposome | 10,0 % |

### Beispiel 6

| Phase A | |
|---|---|
| Perfluordekalin | q.s |
| TiO₂ | 8 % |
| Melanin | 1 % |

| Phase B | |
|---|---|
| Ethanol | q.s |
| Schellack | 1,5 % |
| Natrium- und Triethanolaminsalze von 2-Phenylbenzimidazol-5-sulfonsäure | 15 % |

| Phase C | |
|---|---|
| Jojobaöl | q.s |
| Isohexadecan | 3 % |
| Oxybenzon | 10 % |
| Octylmethoxycinnamat | 15 % |
| Melanin löslich | 3 % |
| Der Anteil der Phasen A, B bzw. C betrug im Gesamtgemisch 35 % , 15 % bzw. 50 %. | |

### Beispiel 7 (Make up mit Selbstbräuner und hohem Lichtschutz)

| Phase A | |
|---|---|
| C₁₂-C₁₅-Alkylbenzoat | q.s. |
| UV-Filter TiO₂ | 5 % |
| Zinkoxid | 1,5 % |
| Kaolin* | 4,5 % |
| pigmentierte Farben für Make up | 2,0 % |
| lösliche Farben je nach Farbton | 1,0 % |
| Melanin | 1,0 % |
| DHA-Liposome gemäß Beispiel 5 | 10 % |

| Phase B | |
|---|---|
| Schellack | 1,0 % |
| Isopropanol | q.s |
| Natrium- und Triethanolaminsalze von 2-Phenylbenzimidazol-5-sulfonsäure | 15 % |

| Phase C | |
|---|---|
| Siliconöl | q.s. |
| Poly(isobutylmethacrylat)-co-methyl FOSEA)-g-Poly(dimethylsiloxan) | 20 % |
| Oxybenzon | 8 % |
| Vitamin E E | 1 % |
| Melanin löslich | 1,5 % |

### Beispiel 8

Es wurden 3 mg einer öligen Phase (auf Basis eines Linolates) pro cm² Haut bei 10 Probanden aufgetragen. Diese Phase enthielt 2 % Octylmethoxycinnamat (OMC) als UV-Filter. Darüber wurden 1 mg/cm² einer wäßrigen Phase aufgetragen. Die wäßrige Phase enthielt 3 % TiO₂, so daß 4 mg/cm² insgesamt 0,14 mg OMC/cm² und 0,2 mg TiOVEIL AQN/cm² aufwiesen. Nach Messung des Lichtschutzfaktors nach dem Diffey-Verfahren ergab sich ein Durchschnittswert von LSF = 7,03.

### Vergleichsbeispiel 1

Es wurde eine Vergleichsemulsion mit 4 mg/cm² Haut bei 10 Probanden aufgetragen. Die Emulsion enthielt 1 % Titaniumdioxid-Filter und 1,5 % des OMC-Filters. Diese Vergleichsemulsion enthielt somit die gleiche Menge UV-Filter wie die beiden Schichten in Beispiel 8 gemeinsam. Nach Messung des Lichtschutzfaktors nach dem Diffey-Verfahren ergab sich ein Durchschnittswert von 4,97.

Der Vergleich zeigt deutlich, daß bereits bei zweischichtiger Anordnung von Lichtschutzmitteln wesentliche Verbesserungen gegenüber den bekannten Emulsionen erreicht werden können.

## Patentansprüche (Patentansprüche für folgende(n) Vertragsstaat(en): AT, BE, CH, LI, DK, ES, FI, FR, GB, GR, IE, IT, LU, MC, NL, PT, SE)

1. Mehrphasiges Lichtschutzmittel, gekennzeichnet durch wenigstens zwei Phasen, die flüssig bis pastenartig oder gel-artig sind, und die sich bei einem kurzen und schonenden Mischvorgang von weniger als 1 Minute und ohne wesentliche Krafteinwirkung spontan innerhalb von Sekunden bis weniger als 10 Minuten voneinander trennen und als getrennte Schichten vorliegen, wobei wenigstens eine Phase einen UV-Filter enthält.

2. Lichtschutzmittel nach Anspruch 1, gekennzeichnet durch wenigstens zwei im wesentlichen flüssige Phasen, die voneinander getrennt vorliegen, wobei wenigstens zwei der Phasen jeweils wenigstens einen UV-Filter enthalten, und sich die Phasen nach einem kurzen und schonenden Mischvorgang spontan trennen unter Verbleiben der jeweiligen UV-Filter in der Phase, in der sie ursprünglich enthalten waren, und die Phasen getrennte Schichten bilden.

3. Lichtschutzmittel nach Anspruch 2, gekennzeichnet durch wenigstens drei im wesentlichen flüssige Phasen, die voneinander getrennt vorliegen, wobei wenigstens zwei der Phasen jeweils einen UV-Filter enthalten, und sich die Phasen nach einem kurzen und schonenden Mischvorgang spontan trennen und getrennte Schichten bilden.

4. Lichtschutzmittel nach einem der Ansprüche 1 bis 3, dadurch gekennzeichnet, daß wenigstens eine der Phasen eine ölige Phase ist, und eine weitere Phasen eine nicht-ölige, ein Perfluorcarbon oder Perfluorcarbongemisch enthaltende Phase ist.

5. Lichtschutzmittel nach Anspruch 4, dadurch gekennzeichnet, daß die ölige Phase ein lipophiles Lichtschutzmittel gelöst oder suspendiert enthält.

6. Lichtschutzmittel nach Anspruch 5, dadurch gekennzeichnet, daß das lipophile Lichtschutzmittel ein UV-Filter ist, ausgewählt aus der Gruppe, die aus Phenylbenzimidazolsulfonsäuresalzen, einem Salicylsäureester, einem Benzophenon, einem Zimtsäureester, einem Benzmalonsäureester, einem Triazin und einem Dibenzoylmenthan-Derivat sowie Dihydroxyaceton besteht, vorzugsweise ausgewählt unter 2-Hydroxy-4-methoxybenzophenon, 2-Ethyl-hexyl-p-methoxycinnamat, Natrium- und Triethanolaminsalzen von 2-Phenylbenzimidazol-5-sulfonsäure, Oxyberizon und Dihydroxyaceton.

7. Lichtschutzmittel nach einem der Ansprüche 1 bis 6, dadurch gekennzeichnet, daß die Phasen kein Wasser enthalten.

8. Lichtschutzmittel nach einem der Ansprüche 1 bis 7, dadurch gekennzeichnet, daß eine nicht-ölige Phase ein hydrophiles Lichtschutzmittel suspendiert oder gelöst enthält.

9. Lichtschutzmittel nach Anspruch 8, dadurch gekennzeichnet, daß der als hydrophiles Lichtschutzmittel eingesetzte UV-Filter aus der Gruppe ausgewählt ist, die aus
Titaniumdioxid, Zinkoxid oder einem Mischoxid (a) von TiO₂ und SiO₂ und/oder Al₂O₃ oder (b) von ZnO und SiO₂ und/oder Al₂O₃ besteht.

10. Lichtschutzmittel nach Anspruch 3, bestehend aus drei Phasen; wobei
die Phase (A) ein oder mehrere Perfluorcarbone und darin verteilt als UV-Filter ein anorganisches Oxid oder Oxidgemisch umfaßt; und
die Phase (B) eine polymere filmbildende Verbindung oder einen Polymeren Lack in einem organischen Lösungsmittel, gegebenenfalls im Gemisch mit einem UV-Filter Umfaßt; und
die Phase (c) wenigstens ein spreitendes Hautöl und wenigstens einen darin enthaltenen lipophilen UV-Filter umfaßt.

11. Lichtschutzmittel nach Anspruch 10, dadurch gekennzeichnet, daß der Anteil der Phasen, bezogen auf die Gesamtzusammensetzung 15 bis 35 Gew-% Phase (A), 5 bis 25 Gew-% Phase (B) und 25 bis 80 Gew-% Phase (C) beträgt.

12. Lichtschutzmittel nach Anspruch 10, dadurch gekennzeichnet, daß das organische Lösungsmittel ein Alkohol ist, insbesondere Isopropanol oder Ethanol.

13. Lichtschutzmittel nach Anspruch 10, dadurch gekennzeichnet, daß der polymere Lack Schellack ist.

14. Lichtschutzmittel nach Anspruch 10, dadurch gekennzeichnet, daß es zusätzlich die Phase (D) umfaßt, bestehend aus einem von Phase (C) verschiedenen öl und gegebenenfalls einem lipophilen UV-Filter.

15. Lichtschutzmittel nach einem der Ansprüche 1 bis 13, dadurch gekennzeichnet, daß die Phasen in einem Dispenser in räumlicher Trennung enthalten sind.

16. Lichtschutzmittel nach Anspruch 15, dadurch gekennzeichnet, daß die Phasen in dem Dispenser in Anteilen Vorliegen, die ihren vorbestimmten Mischungsverhältnissen entsprechen.

17. Kosmetisches verfahren zum Auftragen eines Lichtschutzmittels auf die Haut, dadurch gekennzeichnet, daß in einem Dispenser räumlich getrennt vorliegende, im wesentlichen flüssige und Wasserfreie Phasen; bestehend aus wenigstens
(1) einer öligen Phase mit einem darin enthältenen UV-Filter,
(2) einer Phase mit einem polymeren Lack oder Filmbildner und gegebenenfalls einem UV-Filter und
(3) einer ein oder mehrere Fluorcarbone und ein UV-Filter enthaltenden Phase unmittelbar vor dem Auftragen in vorbestimmten Mischungsverhältnissen miteinander vermischt und in gemischter Form auf die Haut aufgetragen und verteilt werden, so daß sich auf der Haut getrennte Schichten bilden

18. Verfahren nach Anspruch 17, dadurch gekennzeichnet, daß in der Phase, die sich als Schicht auf der Haut bildet und mit dieser unmittelbar in Kontakt kommt, als UV-Filter ein oder mehrere anorganische Pigmente eingearbeitet sind.

19. Verwendung eines Lichtschutzmittels aus wenigstens zwei oder drei flüssigen Phasen, die sich bei kurzzeitigem und schonendem Mischen miteinander im wesentlichen nicht mischen; zur Herstellung einer Schutzschicht auf der Haut gegen UVA-und/oder UVB-Strahlung durch Auftragen von getrennt vorliegenden und unmittelbar vor der Verwendung vermischten Einzelphasen des Lichtschutzmittels, wobei wenigstens zwei Phasen unterschiedliche UV-Filter enthalten, und Verreiben des Gemisches auf der Haut, wobei sich getrennte Schichten auf der Haut bilden.

20. Verwendung nach Anspruch 19, dadurch gekennzeichnet, daß die mit der Haut direkt in Kontakt kommende Phase, die sich als Schicht auf der Haut bildet, als UV-Filter ein oder mehrere anorganische Pigmente enthält.

## Patentansprüche (Patentansprüche für folgende(n) Vertragsstaat(en): DE)

1. Mehrphasiges Lichtschutzmittel, gekennzeichnet durch wenigstens zwei Phasen, die flüssig bis pastenartig oder gel-artig sind, und die'sich bei einem kurzen und schonenden Mischvorgang von weniger als 1 Minute und ohne wesentliche Krafteinwirkung spontan innerhalb von Sekunden bis weniger als 10 Minuten voneinander trennen und als getrennte Schichten vorliegen, wobei wenigstens eine Phase einen UV-Filter enthält und wobei wenigstens eine der Phasen eine ölige Phase ist, und eine weitere Phase eine nicht-ölige, ein Perfluorcarbon oder Perfluorcarbongemisch enthaltende Phase ist.

2. Lichtschutzmittel nach Anspruch 1, gekennzeichnet durch wenigstens zwei im wesentlichen flüssige Phasen, die voneinander getrennt vorliegen, wobei wenigstens zwei der Phasen jeweils wenigstens einen UV-Filter enthalten, und sich die Phasen nach einem kurzen und schonenden Mischvorgang spontan trennen unter Verbleiben der jeweiligen UV-Filter in der Phase, in der sie ursprünglich enthalten waren, und die Phasen getrennte Schichten bilden

3. Lichtschutzmittel nach Anspruch 2, gekennzeichnet durch wenigstens drei im wesentlichen flüssige Phasen, die voneinander getrennt vorliegen, wobei wenigstens zwei der Phasen jeweils einen UV-Filter enthalten, und sich die Phasen nach einem kurzen und schonenden Mischvorgang spontan trennen und getrennte Schichten bilden.

4. Lichtschutzmittel nach Anspruch 1, dadurch gekennzeichnet, daß die ölige Phase ein lipophiles Lichtschutzmittel gelöst oder suspendiert enthält.

5. Lichtschutzmittel nach Anspruch 4, dadurch gekennzeichnet, daß das lipophile Lichtschutzmittel ein UV-Filter ist, ausgewählt aus der Gruppe; die aus Phenylbenzimidazolsulfonsäuresalzen, einem Salicylsäureester, einem Benzophenon, einem Zimtsäureester, einem Benzmalonsäureester, einem Triazin und einem Dibenzoylmenthan-Derivat sowie Dihydroxyaceton besteht, vorzugsweise ausgewählt unter 2-Hydroxy-4-methoxybenzophenon, 2-Ethyl-hexyl-p-methoxycinnamat, Natrium- und Triethanolaminsalzen von 2-Phenylbenzimidazol-5-sulfonsäure, Oxybenzon und Dihydroxyaceton.

6. Lichtschutzmittel nach einem der Ansprüche 1 bis 5, dadurch gekennzeichnet, daß die Phasen kein Wasser enthalten.

7. Lichtschutzmittel nach einem der Ansprüche 1 bis 6, dadurch gekennzeichnet, daß eine nicht-ölige Phase ein hydrophiles Lichtschutzmittel suspendiert oder gelöst enthält.

8. Lichtschutzmittel nach Anspruch 7, dadurch gekennzeichnet, daß der als hydrophiles Lichtschutzmittel eingesetzte UV-Filter aus der Gruppe ausgewählt ist, die aus
Titaniumdioxid, Zinkoxid oder einem Mischoxid (a) von TiO₂ und SiO₂ und/oder Al₂O₃ oder (b) von ZnO und SiO₂ und/oder Al₂O₃ besteht.

9. Lichtschutzmittel nach Anspruch 3, bestehend aus drei Phasen, wobei
die Phase (A) ein oder mehrere Perfluorcarbone und darin verteilt als UV-Filter ein anorganisches Oxid oder Oxidgemisch umfaßt; und
die Phase (B) eine polymere filmbildende Verbindung oder einen polymeren Lack in einem organischen Lösungsmittel, gegebenenfalls im Gemisch mit einem UV-Filter umfaßt; und
die Phase (C) wenigstens ein spreitendes Hautöl und wenigstens einen darin enthaltenen lipophilen UV-Filter umfaßt.

10. Lichtschutzmittel nach Anspruch 9, dadurch gekennzeichnet, daß der Anteil der Phasen, bezogen auf die Gesamtzusammensetzung 15 bis 35 Gew-% Phase (A), 5 bis 25 Gew-% Phase (B) und 25 bis 80 Gew-% Phase (C) beträgt.

11. Lichtschutzmittel nach Anspruch 9, dadurch gekennzeichnet, daß das organische Lösungsmittel ein Alkohol ist, insbesondere Isopropanol oder Ethanol.

12. Lichtschutzmittel nach Anspruch 9, dadurch gekennzeichnet, daß der polymere Lack Schellack ist.

13. Lichtschutzmittel nach Anspruch 9, dadurch gekennzeichnet, daß es zusätzlich die Phase (D) umfaßt, bestehend aus einem von Phase (C) verschiedenen öl und gegebenenfalls einem lipophilen UV-Filter.

14. Lichtschutzmittel nach einem der Ansprüche 1 bis 12, dadurch gekennzeichnet, daß die Phasen in einem Dispenser in räumlicher Trennung enthalten sind.

15. Lichtschutzmittel nach Anspruch 14, dadurch gekennzeichnet, daß die Phasen in dem Dispenser in Anteilen vorliegen, die ihren vorbestimmten Mischungsverhältnissen entsprechen.

16. Kosmetisches Verfahren zum Auftragen eines Lichtschutzmittels auf die Haut, dadurch gekennzeichnet, daß in einem Dispenser räumlich getrennt vorliegende, im wesentlichen flüssige und wasserfreie Phasen, bestehend aus wenigstens
(1) einer öligen Phase mit einem darin enthaltenen UV-Filter,
(2) einer Phase mit einem polymeren Lack oder Filmbildner und gegebenenfalls einem UV-Filter und
(3) einer ein oder mehrere Fluorcarbone und ein UV-Filter enthaltenden Phase unmittelbar vor dem Auftragen in vorbestimmten Mischungsverhältnissen miteinander vermischt und in gemischter Form auf die Haut aufgetragen und verteilt werden, so daß sich auf der Haut getrennte Schichten bilden.

17. Verfahren nach Anspruch 16, dadurch gekennzeichnet, daß in der Phase, die sich als Schicht auf der Haut bildet und mit dieser unmittelbar in Kontakt kommt, als UV-Filter ein oder mehrere anorganische Pigmente eingearbeitet sind.

18. Verwendung eines Lichtschutzmittels aus wenigstens zwei oder drei flüssigen Phasen, die sich bei kurzzeitigem und schonendem Mischen miteinander im wesentlichen nicht mischen, zur Herstellung einer Schutzschicht auf der Haut gegen UVA-und/oder UVB-Strahlung durch Auftragen von getrennt vorliegenden und unmittelbar vor der Verwendung vermischten Einzelphasen des Lichtschutzmittels, wobei wenigstens zwei Phasen unterschiedliche UV-Filter enthalten und wenigstens eine Phase ein Perfluorcarbon oder Perfluorcarbongemisch enthält, und Verreiben des Gemisches auf der Haut, wobei sich getrennte Schichten auf der Haut bilden.

19. Verwendung nach Anspruch 18, dadurch gekennzeichnet, daß die mit der Haut direkt in Kontakt kommende Phase, die sich als Schicht auf der Haut bildet, als UV-Filter ein oder mehrere anorganische Pigmente enthält.

## Claims (Claims for the following Contracting State(s): AT, BE, CH, LI, DK, ES, FI, FR, GB, GR, IE, IT, LU, MC, NL, PT, SE)

1. A multi-phase sunscreen agent, characterized by at least two phases that are liquid to pasty or gelatinous and separate from each other spontaneously within seconds to less than ten minutes after a brief and gentle mixing process lasting less than one minute without any essential application of force, where at least one phase contains a UV filter.

2. A sunscreen agent according to Claim 1, characterized by at least two essentially liquid phases that are separate from each other, where at least two of the phases contain at least one UV filter each, and the phases separate spontaneously after a brief and gentle mixing process, leaving the respective UV filters in the phases in which they were originally present, and the phases form separate layers.

3. A sunscreen agent according to Claim 2, characterized by at least three essentially liquid phases that are separate from each other, where at least two of the phases contain one UV filter each, and the phases separate spontaneously after a brief and gentle mixing process and form separate layers.

4. A sunscreen agent according to one of Claims 1 to 3, characterized in that at least one of the phases is an oily phase, and another phase is a non-oily phase containing a perfluorocarbon or a perfluorocarbon mixture.

5. A sunscreen agent according to Claim 4, characterized in that the oily phase contains a dissolved or suspended lipophilic sunscreen agent.

6. A sunscreen agent according to Claim 5, characterized in that the lipophilic sunscreen agent is a UV filter selected from the group consisting of phenylbenzimidazolesulfonic acid salts, a salicylic acid ester, a benzophenone, a cinnamic acid ester, a benzomalonic acid ester, a triazine and a dibenzoylmenthane derivative as well as dihydroxyacetone, preferably selected from 2-hydroxy-4-methoxybenzophenone, 2-ethylhexyl p-methoxycinnamate, sodium and triethanolamine salts of 2-phenylbenzimidazole-5-sulfonic acid, oxybenzone and dihydroxyacetone.

7. A sunscreen agent according to one of Claims 1 to 6, characterized in that the phases do not contain any water.

8. A sunscreen agent according to one of Claims 1 to 7, characterized in that a non-oily phase contains a suspended or dissolved hydrophilic sunscreen agent.

9. A sunscreen agent according to Claim 8, characterized in that the UV filters used as the hydrophilic sunscreen agent are selected from the group consisting of titanium dioxide, zinc oxide or a mixed oxide (a) of TiO₂ and SiO₂ and/or Al₂O₃ or (b) of ZnO and SiO₂ and/or Al₂O₃.

10. A sunscreen agent according to Claim 3, consisting of three phases, where
phase (A) comprises one or more perfluorocarbons and an inorganic oxide or oxide mixture distributed in it as the UV filter; and
phase (B) comprises a polymer film-forming compound or a polymer lac in an organic solvent, optionally in mixture with a UV filter; and
phase (C) comprises at least one spreading skin oil and at least one lipophilic UV filter contained in it.

11. A sunscreen agent according to Claim 10, characterized in that the proportion of phases, based on the total composition, is 15 to 35 wt% phase (A), 5 to 25 wt% phase (B) and 25 to 80 wt% phase (C).

12. A sunscreen agent according to Claim 10, characterized in that the organic solvent is an alcohol, in particular isopropanol or ethanol.

13. A sunscreen agent according to Claim 10, characterized in that the polymer lac is shellac.

14. A sunscreen agent according to Claim 10, characterized in that it also includes phase (D) consisting of an oil different from phase (C) and optionally a lipophilic UV filter.

15. A sunscreen agent according to one of Claims 1 to 13, characterized in that the phases are contained in a dispenser in which they are spatially separated.

16. A sunscreen agent according to Claim 15, characterized in that the phases are present in the dispenser in proportions that correspond to their predetermined mixing ratios.

17. A cosmetic process for applying a sunscreen agent to the skin, characterized in that essentially liquid and anhydrous phases which are spatially separated in a dispenser and consist of at least:
(1) an oily phase containing a UV filter,
(2) a phase with a polymer lac or film-forming agent and optionally a UV filter, and
(3) a phase containing one or more fluorocarbons and a UV filter are mixed together in predetermined mixing ratios immediately before application and are applied to the skin in mixed form and distributed there, so that separate layers are formed on the skin.

18. A process according to Claim 17, characterized in that one or more inorganic pigments are incorporated as UV filter(s) into the phase which forms a layer on the skin and comes in direct contact with the skin.

19. The use of a sunscreen agent consisting of at least two or three liquid phases that are essentially not miscible with each other when mixed briefly and gently, for preparing a protective layer against UVA and/or UVB radiation on the skin by applying individual phases of the sunscreen agent that are separate from each other and are mixed immediately before use, where at least two phases contain different UV filters, and the mixture is rubbed on the skin, forming separate layers on the skin.

20. The use according to Claim 19, characterized in that the phase which is the layer that forms on the skin and comes in direct contact with the skin contains one or more inorganic pigments as UV filter(s).

## Claims (Claims for the following Contracting State(s): DE)

1. A multi-phase sunscreen agent, characterized by at least two phases that are liquid to pasty or gelatinous and separate from each other spontaneously within seconds to less than ten minutes after a brief and gentle mixing process lasting less than one minute without any essential application of force, where at least one phase contains a UV filter, and wherein at least one of the phases is an oily phase, and another phase is a non-oily phase containing a perfluorocarbon or a perfluorocarbon mixture.

2. A sunscreen agent according to Claim 1, characterized by at least two essentially liquid phases that are separate from each other, where at least two of the phases contain at least one UV filter each, and the phases separate spontaneously after a brief and gentle mixing process, leaving the respective UV filters in the phases in which they were originally present, and the phases form separate layers.

3. A sunscreen agent according to Claim 2, characterized by at least three essentially liquid phases that are separate from each other, where at least two of the phases contain one UV filter each, and the phases separate spontaneously after a brief and gentle mixing process and form separate layers.

4. A sunscreen agent according to Claim 1, characterized in that the oily phase contains a dissolved or suspended lipophilic sunscreen agent.

5. A sunscreen agent according to Claim 4, characterized in that the lipophilic sunscreen agent is a UV filter selected from the group consisting of phenylbenzimidazolesulfonic acid salts, a salicylic acid ester, a benzophenone, a cinnamic acid ester, a benzomalonic acid ester, a triazine and a dibenzoylmenthane derivative as well as dihydroxyacetone, preferably selected from 2-hydroxy-4-methoxybenzophenone, 2-ethylhexyl p-methoxycinnamate, sodium and triethanolamine salts of 2-phenylbenzimidazole-5-sulfonic acid, oxybenzone and dihydroxyacetone.

6. A sunscreen agent according to one of Claims 1 to 5, characterized in that the phases do not contain any water.

7. A sunscreen agent according to one of Claims 1 to 6, characterized in that a non-oily phase contains a suspended or dissolved hydrophilic sunscreen agent.

8. A sunscreen agent according to Claim 7, characterized in that the UV filters used as the hydrophilic sunscreen agent are selected from the group consisting of titanium dioxide, zinc oxide or a mixed oxide (a) of TiO₂ and SiO₂ and/or Al₂O₃ or (b) of ZnO and SiO₂ and/or Al₂O₃.

9. A sunscreen agent according to Claim 3, consisting of three phases, where
phase (A) comprises one or more perfluorocarbons and an inorganic oxide or oxide mixture distributed in it as the UV filter; and
phase (B) comprises a polymer film-forming compound or a polymer lac in an organic solvent, optionally in mixture with a UV filter; and
phase (C) comprises at least one spreading skin oil and at least one lipophilic UV filter contained in it.

10. A sunscreen agent according to Claim 9, characterized in that the proportion of phases, based on the total composition, is 15 to 35 wt% phase (A), 5 to 25 wt% phase (B) and 25 to 80 wt% phase (C).

11. A sunscreen agent according to Claim 9, characterized in that the organic solvent is an alcohol, in particular isopropanol or ethanol.

12. A sunscreen agent according to Claim 9, characterized in that the polymer lac is shellac.

13. A sunscreen agent according to Claim 9, characterized in that it also includes phase (D) consisting of an oil different from phase (C) and optionally a lipophilic UV filter.

14. A sunscreen agent according to one of Claims 1 to 12, characterized in that the phases are contained in a dispenser in which they are spatially separated.

15. A sunscreen agent according to Claim 14, characterized in that the phases are present in the dispenser in proportions that correspond to their predetermined mixing ratios.

16. A cosmetic process for applying a sunscreen agent to the skin, characterized in that essentially liquid and anhydrous phases which are spatially separated in a dispenser and consist of at least:
(1) an oily phase containing a UV filter,
(2) a phase with a polymer lac or film-forming agent and optionally a UV filter, and
(3) a phase containing one or more fluorocarbons and a UV filter are mixed together in predetermined mixing ratios immediately before application and are applied to the skin in mixed form and distributed there, so that separate layers are formed on the skin.

17. A process according to Claim 16, characterized in that one or more inorganic pigments are incorporated as UV filter(s) into the phase which forms a layer on the skin and comes in direct contact with the skin.

18. The use of a sunscreen agent consisting of at least two or three liquid phases that are essentially not miscible with each other when mixed briefly and gently, for preparing a protective layer against UVA and/or UVB radiation on the skin by applying individual phases of the sunscreen agent that are separate from each other and are mixed immediately before use, where at least two phases contain different UV filters and at least one phase contains a perfluorocarbon or a perfluorocarbon mixture, and the mixture is rubbed on the skin, forming separate layers on the skin.

19. The use according to Claim 18, characterized in that the phase which is the layer that forms on the skin and comes in direct contact with the skin contains one or more inorganic pigments as UV filter(s).

## Revendications (Revendications pour l'(les) Etat(s) contractant(s) suivant(s): AT, BE, CH, LI, DK, ES, FI, FR, GB, GR, IE, IT, LU, MC, NL, PT, SE)

1. Agent photoprotecteur à plusieurs phases, caractérisé par au moins deux phases liquides à pâteuses ou du genre gel, qui se séparent spontanément l'une de l'autre en l'espace de quelques secondes à moins de 10 minutes au terme d'un processus de mélange bref et délicat de moins d'l minute et sans application d'efforts notables et se présentent sous forme de couches distinctes, une phase au moins contenant un filtre UV.

2. Agent photoprotecteur selon la revendication 1, caractérisé par au moins deux phases sensiblement liquides se présentant séparément l'une de l'autre, deux des phases, au moins, contenant chacune au moins un filtre UV, les phases se séparant spontanément au terme d'un processus de mélange bref et délicat, en laissant subsister les filtres UV respectifs dans la phase dans laquelle ils étaient compris initialement, et les phases formant des couches distinctes.

3. Agent photoprotecteur selon la revendication 2, caractérisé par au moins trois phases sensiblement liquides se présentant séparément l'une de l'autre, deux au moins desdites phases comportant chacune un filtre UV, lesdites phases se séparant spontanément à l'issue d'un processus de mélange bref et délicat et formant des couches distinctes.

4. Agent photoprotecteur selon l'une des revendications 1 à 3, caractérisé en ce qu'au moins une des phases est une phase huileuse et une autre phase est une phase non-huileuse contenant un perfluorocarbone ou un mélange de perfluorocarbones.

5. Agent photoprotecteur selon la revendication 4, caractérisé en ce que la phase huileuse contient un agent photoprotecteur lipophile à l'état dissous ou en suspension.

6. Agent photoprotecteur selon la revendication 5, caractérisé en ce que l'agent photoprotecteur lipophile est un filtre UV, sélectionné dans le groupe comprenant des sels d'acide phénylbenzimidazolsulphonique, un ester d'acide salicylique, une benzophénone, un ester d'acide de cannelle, un ester d'acide benzomalonique, une triazine et un dérivé de dibenzoylmenthane et de la dihydroxyacétone, de préférence sélectionnée parmi l'hydroxy-2 méthoxy-4 benzophénone, l'éthyl-2 p-hexylméthoxycinnamate, des sels de sodium et de triéthanolamine d'acide phényl-2 benzimidazole-5 sulphonique, l'oxybenzone ou la dihydroxyacétone.

7. Agent photoprotecteur selon l'une des revendications 1 à 6, caractérisé en ce que les phases ne renferment pas d'eau.

8. Agent photoprotecteur selon l'une des revendications 1 à 7, caractérisé en ce qu'une phase non-huileuse contient un agent photoprotecteur hydrophile en suspension ou dissous.

9. Agent photoprotecteur selon la revendication 8, caractérisé en ce que le filtre UV utilisé comme agent photoprotecteur hydrophile est sélectionné dans le groupe comprenant le dioxyde de titane, l'oxyde de zinc ou un oxyde mixte (a) de TiO₂ et SiO₂ et/ou Al₂O₃ ou (b) de ZnO et SiO₂ et/ou Al₂O₃.

10. Agent photoprotecteur selon la revendication 3, comprenant trois phases,
la phase (A) contenant un ou plusieurs perfluorocarbones et, distribué dans ceux-ci en tant que filtre UV, un oxyde minéral ou un mélange d'oxydes minéral; et
la phase (B) contenant un composé polymère filmogène ou une laque polymère dans un solvant organique, le cas échéant en mélange avec un filtre UV; et
la phase (C) comprenant au moins une huile dispersive agissant sur l'épiderme et au moins un filtre UV lipophile présent dans cette dernière.

11. Agent photoprotecteur selon la revendication 10, caractérisé en ce que la part des phases, rapportée à la composition globale, est de 15 à 35 % en poids de phase (A), de 5 à 25 % en poids de phase (B) et de 25 à 80 % en poids de phase (C).

12. Agent photoprotecteur selon la revendication 10, caractérisé en ce que le solvant organique est un alcool, en particulier de l'isopropanol ou de l'éthanol.

13. Agent photoprotecteur selon la revendication 10, caractérisé en ce que la laque polymère est de la gomme laque.

14. Agent photoprotecteur selon la revendication 10, caractérisé en ce qu'il comprend encore la phase (D) consistant en une huile différente de la phase (C) et le cas échéant d'un filtre UV lipophile.

15. Agent photoprotecteur selon l'une des revendications 1 à 13, caractérisé en ce que les phases sont contenues dans un dispensateur en étant séparées spatialement.

16. Agent photoprotecteur selon la revendication 15, caractérisé en ce que les phases présentes dans le dispensateur se présentent en fractions qui correspondent à leurs rapports de mélange déterminés préalablement.

17. Procédé cosmétique d'application d'un agent photoprotecteur sur la peau, caractérisé en ce que des phases spatialement séparées dans un dispensateur et sensiblement liquides et exemptes d'eau, comprenant au moins
(1) une phase huileuse, avec un filtre UV contenu dans cette dernière,
(2) une phase avec une laque polymère ou un agent filmogène et le cas échéant un filtre UV, et
(3) une phase renfermant un ou plusieurs fluorocarbones et un filtre UV, sont mélangées les unes avec les autres immédiatement avant l'application suivant des rapports de mélange déterminés préalablement, et appliquées et réparties sur la peau sous forme mélangée, de façon à ce qu'il se forme des couches distinctes sur la peau.

18. Procédé selon la revendication 17, caractérisé en ce qu'on incorpore un ou plusieurs pigments minéraux devant servir de filtres UV dans la phase qui se forme en couche sur la peau et qui vient directement en contact avec cette dernière.

19. Utilisation d'un agent photoprotecteur comprenant au moins deux ou trois phases liquides qui, après un mélange de brève durée et délicat, ne se mélangent pratiquement pas les unes aux autres, pour créer sur la peau une couche protectrice contre le rayonnement UVA et/ou UVB, par application de phases individuelles de l'agent photoprotecteur se présentant séparément et mélangées immédiatement avant l'utilisation, au moins deux phases contenant des filtres UV différents, et étalement du mélange sur la peau par frottement, à la suite de quoi deux couches distinctes se forment sur la peau.

20. Utilisation selon la revendication 19, caractérisée en ce que la phase venant directement en contact avec la peau et se présentant sous forme de couche sur la peau contient un ou plusieurs pigments minéraux en tant que filtres UV.

## Revendications (Revendications pour l'(les) Etat(s) contractant(s) suivant(s): DE)

1. Agent photoprotecteur à plusieurs phases, caractérisé par au moins deux phases liquides à pâteuses ou du genre gel, qui se séparent spontanément l'une de l'autre en l'espace de quelques secondes à moins de 10 minutes au terme d'un processus de mélange bref et délicat de moins d'l minute et sans application d'efforts notables et se présentent sous forme de couches distinctes, une phase au moins contenant un filtre UV, et une autre phase est une phase non-huileuse contenent un perfluorocarbone ou un mélange de perfluorocarbones.

2. Agent photoprotecteur selon la revendication 1, caractérisé par au moins deux phases sensiblement liquides se présentant séparément l'une de l'autre, deux des phases, au moins, contenant chacune au moins un filtre UV, les phases se séparant spontanément au terme d'un processus de mélange bref et délicat, en laissant subsister les filtres UV respectifs dans la phase dans laquelle ils étaient compris initialement, et les phases formant des couches distinctes.

3. Agent photoprotecteur selon la revendication 2, caractérisé par au moins trois phases sensiblement liquides se présentant séparément l'une de l'autre, deux au moins desdites phases comportant chacune un filtre UV, lesdites phases se séparant spontanément à l'issue d'un processus de mélange bref et délicat et formant des couches distinctes.

4. Agent photoprotecteur selon la revendication 1, caractérisé en ce que la phase huileuse contient un agent photoprotecteur lipophile à l'état dissous ou en suspension.

5. Agent photoprotecteur selon la revendication 4, caractérisé en ce que l'agent photoprotecteur lipophile est un filtre UV, sélectionné dans le groupe comprenant des sels d'acide phénylbenzimidazolsulphonique, un ester d'acide salicylique, une benzophénone, un ester d'acide de cannelle, un ester d'acide benzomalonique, une triazine et un dérivé de dibenzoylmenthane et de la dihydroxyacétone, de préférence sélectionnée parmi l'hydroxy-2 méthoxy-4 benzophénone, l'éthyl-2 p-hexylméthoxycinnamate, des sels de sodium et de triéthanolamine d'acide phényl-2 benzimidazole-5 sulphonique, l'oxybenzone ou la dihydroxyacétone.

6. Agent photoprotecteur selon l'une des revendications 1 à 5, caractérisé en ce que les phases ne renferment pas d'eau.

7. Agent photoprotecteur selon l'une des revendications 1 à 6, caractérisé en ce qu'une phase non-huileuse contient un agent photoprotecteur hydrophile en suspension ou dissous.

8. Agent photoprotecteur selon la revendication 7, caractérisé en ce que le filtre UV utilisé comme agent photoprotecteur hydrophile est sélectionné dans le groupe comprenant le dioxyde de titane, l'oxyde de zinc ou un oxyde mixte (a) de TiO₂ et SiO₂ et/ou Al₂O₃ ou (b) de ZnO et SiO₂ et/ou Al₂O₃.

9. Agent photoprotecteur selon la revendication 3, comprenant trois phases,
la phase (A) contenant un ou plusieurs perfluorocarbones et, distribué dans ceux-ci en tant que filtre UV, un oxyde minéral ou un mélange d'oxydes minéral; et
la phase (B) contenant un composé polymère filmogène ou une laque polymère dans un solvant organique, le cas échéant en mélange avec un filtre UV; et
la phase (C) comprenant au moins une huile dispersive agissant sur l'épiderme et au moins un filtre UV lipophile présent dans cette dernière.

10. Agent photoprotecteur selon la revendication 9, caractérisé en ce que la part des phases, rapportée à la composition globale, est de 15 à 35 % en poids de phase (A), de 5 à 25 % en poids de phase (B) et de 25 à 80 % en poids de phase (C).

11. Agent photoprotecteur selon la revendication 9, caractérisé en ce que le solvant organique est un alcool, en particulier de l'isopropanol ou de l'éthanol.

12. Agent photoprotecteur selon la revendication 9, caractérisé en ce que la laque polymère est de la gomme laque.

13. Agent photoprotecteur selon la revendication 9, caractérisé en ce qu'il comprend encore la phase (D) consistant en une huile différente de la phase (C) et le cas échéant d'un filtre UV lipophile.

14. Agent photoprotecteur selon l'une des revendications 1 à 12, caractérisé en ce que les phases sont contenues dans un dispensateur en étant séparées spatialement.

15. Agent photoprotecteur selon la revendication 14, caractérisé en ce que les phases présentes dans le dispensateur se présentent en fractions qui correspondent à leurs rapports de mélange déterminés préalablement.

16. Procédé cosmétique d'application d'un agent photoprotecteur sur la peau, caractérisé en ce que des phases spatialement séparées dans un dispensateur et sensiblement liquides et exemptes d'eau, comprenant au moins
(1) une phase huileuse, avec un filtre UV contenu dans cette dernière,
(2) une phase avec une laque polymère ou un agent filmogène et le cas échéant un filtre UV, et
(3) une phase renfermant un ou plusieurs fluorocarbones et un filtre UV, sont mélangées les unes avec les autres immédiatement avant l'application suivant des rapports de mélange déterminés préalablement, et appliquées et réparties sur la peau sous forme mélangée, de façon à ce qu'il se forme des couches distinctes sur la peau.

17. Procédé selon la revendication 16, caractérisé en ce qu'on incorpore un ou plusieurs pigments minéraux devant servir de filtres UV dans la phase qui se forme en couche sur la peau et qui vient directement en contact avec cette dernière.

18. Utilisation d'un agent photoprotecteur comprenant au moins deux ou trois phases liquides qui, après un mélange de brève durée et délicat, ne se mélangent pratiquement pas les unes aux autres, pour créer sur la peau une couche protectrice contre le rayonnement UVA et/ou UVB, par application de phases individuelles de l'agent photoprotecteur se présentant séparément et mélangées immédiatement avant l'utilisation, au moins deux phases contenant des filtres UV différents, au moins une phase contenant un perfluorocarbon ou un mélange de perfluorocarbones, et étalement du mélange sur la peau par frottement, à la suite de quoi deux couches distinctes se forment sur la peau.

19. Utilisation selon la revendication 18, caractérisée en ce que la phase venant directement en contact avec la peau et se présentant sous forme de couche sur la peau contient un ou plusieurs pigments minéraux en tant que filtres UV.
